# FASCICULE DE BREVET EUROPEEN

(11) **EP 1 233 731 B1**
(45) Date de publication et mention de la délivrance du brevet: **29.12.2004**
(21) Numéro de dépôt: 00979740.8
(22) Date de dépôt: 15.11.2000
(51) Int. Cl.: A61F 2/24, A61M 25/00, A61B 17/02

(54) **DISPOSITIF DE REMPLACEMENT D'UNE VALVE CARDIAQUE PAR VOIE PERCUTANEE**
PERKUTANE VORRICHTUNG ZUM ERSETZEN EINER HERZKLAPPE
DEVICE FOR REPLACING A CARDIAC VALVE BY PERCUTANEOUS ROUTE

(30) Priorité: 17.11.1999 FR 9914462
(43) Date de publication de la demande: 28.08.2002
(73) Titulaire: Seguin, Jacques, 75009 Paris (FR)
(72) Inventeur: Seguin, Jacques, 75009 Paris (FR)
(86) Numéro de dépôt international: PCT/FR2000/003176
(87) Numéro de publication internationale: WO 2001/035870

(56) Documents cités:
- WO-A-99/12483
- WO-A-99/15227
- US-A- 5 156 610
- US-A- 5 370 647
- US-A- 5 924 424
- US-A- 5 968 064

## Description

La présente invention concerne un dispositif de remplacement d'une valve cardiaque par voie percutanée.

Le remplacement d'une valve cardiaque défectueuse est actuellement, le plus fréquemment, réalisé par ouverture du thorax, mise du patient sous circulation extra-corporelle, arrêt cardiaque temporaire, et ouverture du coeur, en vue de l'exérèse et du remplacement de la valve.

Ces étapes successives de l'opération ont pour inconvénient d'impliquer une hospitalisation relativement longue du patient, et d'être complexe et coûteuse.

Pour remédier à cet inconvénient, il a été envisagé de remplacer une valve cardiaque par voie peu invasive. Les demandes internationales (PCT) n° WO 93/01768 et WO 97/28807, ainsi que les brevets américains n° 5 814 097, 5 370 685 ou 5 545 214 illustrent des techniques peu invasives connues ainsi que des instruments de mise en oeuvre de ces techniques. Le document US 5 924 424 décrit un dispositif selon le préambule de la revendication 1.

Les techniques existantes sont toutefois considérées comme n'étant pas parfaitement satisfaisantes et comme susceptibles d'être améliorées. En particulier, ces techniques ont pour inconvénient d'imposer en tout état de cause la mise du patient sous circulation extra-corporelle et l'arrêt temporaire du coeur ; elles sont difficiles à mettre en pratique ; elles ne permettent pas un contrôle précis du diamètre selon lequel la valve native est coupée, en vue du calibrage ultérieur de la valve prothétique ; elles entraînent des risques de diffusion de fragments de valve native, souvent calcifiée, dans l'organisme, qui peuvent conduire à une embolie, ainsi que des risques de perforation de la paroi aortique ou cardiaque ; elles induisent en outre des risques de reflux aigus du sang lors de l'ablation de la valve native.

Le dispositif selon la présente invention a été conçu dans le but de pallier les insuffisances de ces techniques.

En particulier, l'invention a pour objectif de fournir un dispositif donnant toute satisfaction en ce qui concerne l'exérèse et le remplacement de la valve, en permettant d'intervenir sans ouverture du thorax, arrêt cardiaque et/ou ouverture du coeur, et de prévenir toute diffusion dans le système circulatoire de fragments de la valve retirée.

Les termes "distal" et "proximal" utilisés ci-après pour exposer l'invention sont définis par rapport au sens d'écoulement du sang.

Le dispositif selon l'invention comprend :
- un élément allongé de support ;
- une première série de lames allongées, disposées autour de la circonférence dudit élément allongé ; ces lames sont reliées de manière pivotante à l'élément allongé au niveau de leurs extrémités longitudinales proximales et présentent chacune un bord tranchant au niveau de leur extrémité longitudinale distale ; ces lames peuvent pivoter par rapport à l'élément allongé entre une position de repliage, dans laquelle elles se trouvent près de la paroi de l'élément allongé de telle sorte qu'elles ne font pas obstacle à l'introduction et au coulissement du dispositif dans le conduit corporel dans lequel se trouve la valve, en particulier dans l'aorte, et une position de dépliage, dans laquelle ces lames sont déployées en corolle de telle sorte que leurs bords tranchants sont placés dans le prolongement les uns des autres et constituent ainsi un bord circulaire tranchant ;
- une deuxième série de lames, disposée consécutivement à ladite première série de lames dans la direction distale ; les lames de cette deuxième série de lames ont une structure identique à celle des lames de ladite première série de lames, sinon que ces lames de cette deuxième série sont reliées à l'élément allongé par leurs extrémités longitudinales distales et présentent chacune un bord tranchant au niveau de leur extrémité longitudinale proximale ;
- des moyens permettant d'amener les lames desdites première et deuxième séries de lames de leur position de repliage à leur position de dépliage ;
- des moyens permettant de déplacer axialement lesdites séries de lames en direction l'une de l'autre, entre une position d'éloignement mutuel de ces séries de lames, dans laquelle une série de lames peut être placée axialement d'un côté de la valve native tandis que l'autre série de lames est placée axialement de l'autre côté de cette valve, et une position de rapprochement, dans laquelle les bords circulaires tranchants de ces deux séries de lames sont amenés en contact mutuel et viennent ainsi sectionner la valve native de manière à la séparer dudit conduit corporel ; et
- des moyens de repérage par voie percutanée de la position axiale du dispositif par rapport à la valve native, permettant de positionner chacune des deux séries de lames précitées d'un côté de cette valve.

Le dispositif selon l'invention peut être introduit par voie percutanée dans ledit conduit corporel et être coulissé dans ce conduit jusqu'à ce que chacune des séries de lames précitées soit placée d'un côté de la valve. Cette position est repérée à l'aide desdits moyens de repérage.

Un système de perfusion périphérique ou de circulation extra-corporelle peut être mis en place pour faciliter l'écoulement du sang, en vue d'empêcher la stagnation du sang dans le coeur.

Après le positionnement précité du dispositif, les lames des deux séries de lames sont déployées, puis ces deux séries sont rapprochées l'une de l'autre jusqu'à sectionner la valve. La conformation de ces lames permet de réaliser cette section en une seule opération, donc sans générer de fragments susceptibles d'être diffusés dans le système circulatoire, ou tout au moins en ne générant que très peu de tels fragments ; cette conformation permet en outre un contrôle précis du diamètre selon lequel la valve native est sectionnée, en vue du calibrage ultérieur de la valve prothétique.

Les lames sont ensuite ramenées en position de repliage.

La valve prothétique est alors mise en place.

Cette valve peut être séparée du dispositif, auquel cas ce dernier est retiré puis la valve prothétique est introduite et positionnée dans ledit conduit corporel au moyen d'un dispositif distinct. De préférence toutefois, le dispositif selon l'invention comprend une valve prothétique proximale, à structure radialement déployable, cette valve prothétique pouvant occuper une position de repliage, dans laquelle elle se trouve près de la paroi dudit élément allongé et ne fait pas obstacle à l'introduction et coulissement du dispositif dans ledit conduit corporel, et une position de dépliage, dans laquelle elle prend appui contre la paroi de ce conduit et est à même de remplacer la valve cardiaque native.

Le dispositif permet ainsi d'introduire et de positionner la valve prothétique à l'endroit adéquat dans le conduit corporel, par le même geste que celui permettant de sectionner la valve native. Après sectionnement de cette dernière, le dispositif est coulissé axialement dans la direction distale afin d'amener la valve prothétique au niveau adéquat dans ce conduit, après quoi cette valve prothétique est déployée. Le dispositif est ensuite retiré et la valve native sectionnée est récupérée.

De préférence, ledit élément allongé de support est un cathéter tubulaire.

Ce cathéter permet ainsi l'écoulement du sang au travers de lui le temps de l'exérèse de la valve native.

La section du conduit de ce cathéter peut être suffisante pour permettre l'écoulement du sang au travers de ce conduit, ce qui limite ou évite le recours à une mise du patient en circulation extra-corporelle. Le cathéter peut aussi avoir un diamètre réduit, ce qui facilite l'introduction et le coulissement du dispositif dans le conduit corporel, mais il est alors nécessaire d'assurer la circulation périphérique par un système externe d'assistance, telle qu'un système de circulation extra-corporelle.

Le cathéter comprend une ouverture distale latérale pour permettre au sang de rejoindre le conduit corporel, par exemple l'aorte ascendante, cette ouverture étant aménagée de manière à ce que la longueur de cathéter traversée par le sang soit la plus courte possible.

De préférence, le dispositif comprend un ballonnet gonflable distal, placé au niveau de la face extérieure dudit élément allongé ; ce ballonnet est conformé pour pouvoir occuper une position de repliage, dans laquelle il présente une section telle qu'il ne fait pas obstacle à l'introduction au coulissement du dispositif dans ledit conduit corporel, et une position de dépliage, dans laquelle il occupe l'ensemble de l'espace existant entre la face extérieure dudit élément allongé et la paroi dudit conduit corporel et vient, par un bord périphérique qu'il comprend, en appui contre cette paroi.

Le ballonnet est gonflé après le positionnement des séries de lames de part et d'autre de la valve native, pour empêcher le reflux du sang lors de l'ablation de la valve native. Lorsque ledit élément allongé est un cathéter, ce ballonnet permet en outre d'amener ce sang à s'écouler uniquement au travers du cathéter.

Une fois la valve prothétique mise en place, le ballonnet est ramené en position de repliage de manière à rétablir le flux sanguin au travers du conduit corporel.

De préférence, le dispositif comprend un filtre distal en matériau souple, placé au niveau de la face extérieure dudit élément allongé ; ce filtre est conformé pour pouvoir occuper une position de repliage, dans laquelle il présente une section telle qu'il ne fait pas obstacle à l'introduction et au coulissement du dispositif dans ledit conduit corporel, et une position de dépliage, dans laquelle il occupe l'ensemble de l'espace existant entre la face extérieure dudit élément allongé et la paroi de ce conduit et vient, par un bord périphérique qu'il comprend, en appui contre cette paroi.

Ce filtre permet de capter les éventuels fragments générés par l'exérèse de la valve, et de les retenir afin qu'ils soient retirés de la circulation sanguine.

Le dispositif peut comprendre des moyens permettant de déplacer lesdites séries de lames dans le sens axial indépendamment dudit ballonnet et/ou dudit filtre. Une fois déployés, ce ou ces derniers n'ont pas à être déplacés axialement dans le conduit corporel lors du déplacement axial précité des séries de lames.

Ledit ballonnet et/ou ledit filtre peuvent également être séparés du dispositif, en étant montés sur un élément allongé de support qui leur est propre.

En cas d'intervention sur une valve mitrale, ce ballonnet et/ou ce filtre sont introduits dans l'aorte par voie artérielle périphérique, et le dispositif est quant à lui introduit dans le coeur par le système veineux périphérique, jusqu'à l'oreillette droite puis dans l'oreillette gauche à travers le septum inter-auriculaire, jusqu'au niveau de la valve mitrale.

La valve prothétique peut avantageusement comprendre une armature en matériau à mémoire de forme, notamment en alliage Nickel-titane connu sous la dénomination "NITINOL".

Cette même valve peut comprendre des valvules en matériau biologique (valvules animales ou humaines conservées), ou des valvules en matériau synthétique, tel qu'en polymère.

Pour sa bonne compréhension, l'invention est à nouveau décrite ci-dessous en référence au dessin schématique annexé représentant, à titre d'exemple non limitatif, une forme de réalisation préférée du dispositif qu'elle concerne.
La figure 1 en est une vue coupe longitudinale, selon une première forme de réalisation destinée au traitement d'une valve aortique ;
la figure 2 en est une vue en coupe transversale, selon la ligne II-II de la figure 1 ;
la figure 3 est une vue similaire à la figure 2, dans une autre position d'un sous-ensemble qu'il comprend ;
la figure 4 est une vue en perspective d'une valve prothétique dont il permet la mise en place ;
les figures 5 à 9 en sont des vues alors qu'il est positionné dans un coeur, au niveau de la valve à traiter, au cours de différentes opérations successives par lesquelles cette valve est sectionnée et la valve prothétique montrée à la figure 4 est mise en place ;
la figure 10 est une vue de la valve prothétique montrée à la figure 4, après mise en place, et
la figure 11 est une vue du dispositif selon une autre forme de réalisation, destinée au traitement d'une valve mitrale.

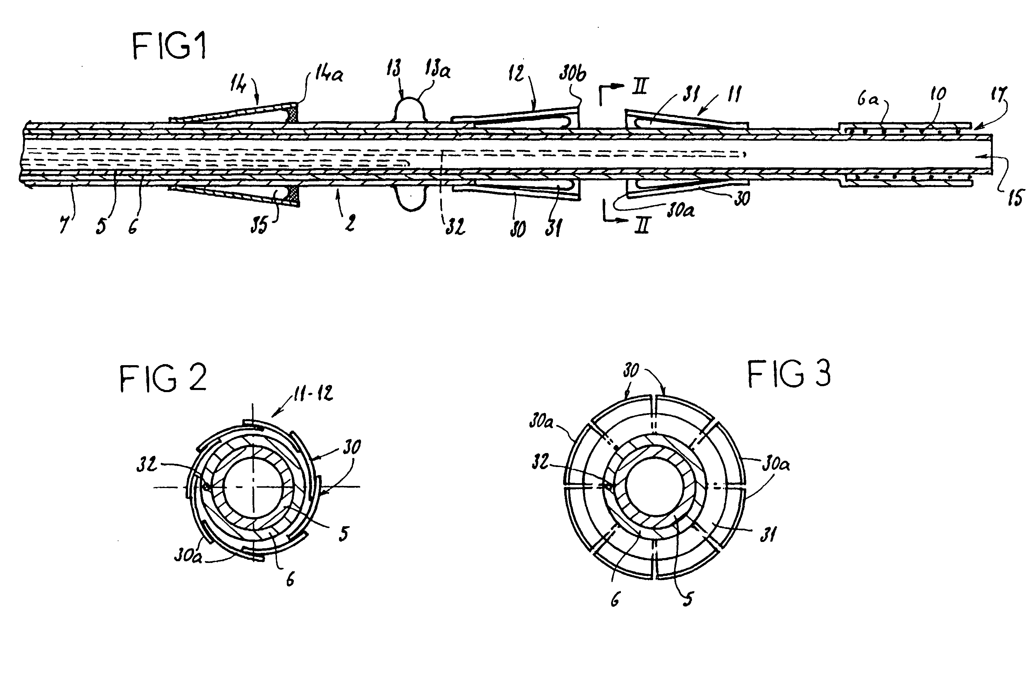

Les figures 1 à 3 représentent un dispositif 1 de remplacement d'une valve cardiaque par voie percutanée.

Ce dispositif comprend un cathéter tubulaire 2 formé de trois tubes 5, 6, 7 engagés les uns dans les autres sur lesquels sont placés, du côté proximal vers le côté distal (considérés par rapport à l'écoulement du sang, c'est-à-dire de la droite vers la gauche de la figure 1), une valve prothétique 10, deux séries de lames 11, 12, un ballonnet 13 et un filtre 14.

Les trois tubes 5, 6, 7 sont montés coulissants les uns dans les autres. Le tube intérieur 5 délimite un conduit 15 dont la section est suffisante pour permettre l'écoulement du sang à travers lui.

Du côté proximal, le tube intermédiaire 6 forme une tulipe 6a délimitant, avec le tube intérieur 5, une cavité annulaire 17 dans laquelle est contenue la valve prothétique 10 à l'état replié.

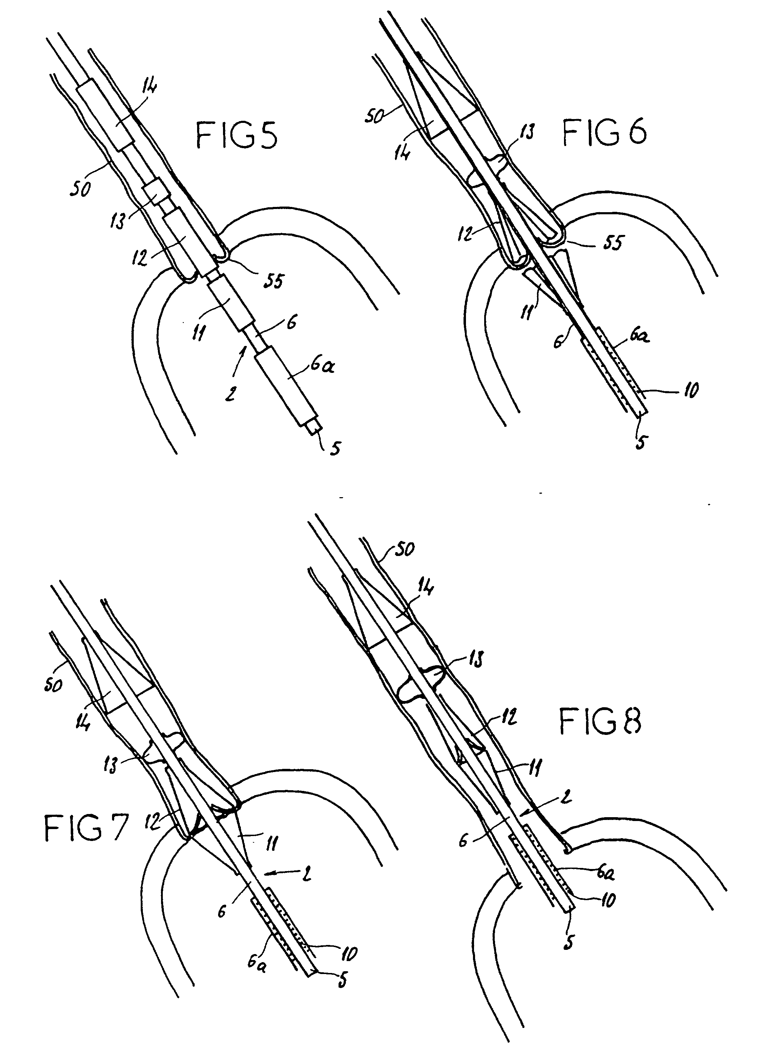

La figure 4 montre que cette valve 10 comprend une armature 20 et des valvules 21 montées de manière fonctionnellement mobiles sur cette armature 20.

L'armature est constituée par assemblage de fils 22, 23, 24 en matériau à mémoire de forme, notamment en alliage Nickel-titane connu sous la dénomination "NITINOL", à savoir :
- un fil 22 d'extrémité proximale, présentant à l'état déployé de la valve 10 une forme sensiblement circulaire ;
- un fil 23 d'extrémité distale formant trois ondulations dans le sens axial, ces ondulations étant régulièrement réparties sur la circonférence de la valve 10, et
- un fil intermédiaire 24 formant des ondulations longitudinales entre les fils 22 et 23, ce fil 24 étant relié à ces derniers par les extrémités de chacune de ces ondulations.

Les valvules 21, quant à elles, sont en matériau biologique (valvules animales ou humaines conservées), ou en matériau synthétique, tel qu'un polymère.

L'armature 20 peut, lorsque son matériau est refroidi, être contractée radialement de telle sorte que la valve 10 peut entrer dans la cavité 17. Lorsque ce matériau est réchauffé à la température de l'organisme, cette armature 20 retrouve sa forme d'origine, représentée à la figure 4, dans laquelle elle a un diamètre adapté à celui du conduit corporel, en particulier l'aorte, dans lequel se trouve la valve native à traiter. Ce diamètre de l'armature 20 est tel que la valve 10 prend appui contre la paroi du conduit corporel et est immobilisée dans le sens axial par rapport à celui-ci.

Chaque série de lame 11, 12 comprend des lames allongées métalliques 30 et un ballonnet gonflable 31 situé entre le cathéter 2 et ces lames 30.

Les lames 30 ont un profil incurvé et sont disposées sur la circonférence du cathéter 2.

Les lames 30 de la série proximale 11 sont reliées de manière pivotante au tube 6 par leurs extrémités proximales et comprennent un bord distal tranchant 30a, tandis que les lames 30 de la série distale 12 sont reliées de manière pivotante au tube extérieur 7 par leurs extrémités distales et comprennent un bord proximal tranchant 30b.

La liaison des lames 30 aux tubes 6 et 7 respectivement, est réalisée par soudage des extrémités des lames 30 entre elles de manière à former un anneau, cet anneau étant fixé axialement au tube 6, 7 correspondant par sertissage de cet anneau sur ce tube 6, 7, le pivotement des lames 30 étant réalisé par simple déformation élastique de ces lames 30.

Ce pivotement est possible entre une position de repliage des lames 30, radialement interne par rapport au cathéter 2, montrée aux figures 1 et 2, et une position de dépliage de ces lames 30, radialement externe par rapport à ce cathéter 2, montrée à la figure 3. Dans la position de repliage, les lames 30 se trouvent près de la paroi du tube 6 et se recouvrent partiellement les unes les autres de telle sorte qu'elles ne font pas obstacle à l'introduction et au coulissement du dispositif 1 dans le conduit corporel dans lequel se trouve la valve native à traiter ; dans ladite position de dépliage, les lames 30 sont déployées en corolle de telle sorte que leurs bords tranchants 30a, 30b sont placés dans le prolongement les uns des autres et constituent ainsi un bord circulaire tranchant, visible sur la figure 3.

Chaque ballonnet 31, placé entre le tube 6 et les lames 30, peut être gonflé depuis l'extrémité du cathéter 2 qui sort du patient, par un conduit 32 aménagé dans le tube 6. Il permet ainsi, lorsqu'il est gonflé, d'amener les lames 30 de leur position de repliage à leur position de dépliage, et inversement lorsqu'il est dégonflé.

Le coulissement axial du tube 6 par rapport au tube 7 permet de déplacer axialement les séries de lames 11, 12 en direction l'une de l'autre, entre des positions d'éloignement mutuel, montrée à la figure 1, et de rapprochement mutuel. Dans la première de ces positions, une série de lames 11 peut être placée axialement d'un côté de la valve native tandis que l'autre série de lames 12 est placée axialement de l'autre côté de cette valve, tandis que dans la deuxième de ces positions, les bords circulaires tranchants de ces deux séries de lames 11, 12 sont amenés en contact mutuel et viennent ainsi sectionner la valve native de manière à la séparer dudit conduit corporel.

Les tubes 5 à 7 comprennent en outre des repères (non visibles sur les figures) au sulfate de baryum, permettant le repérage par voie percutanée de la position axiale du dispositif 1 par rapport à la valve native, afin que chacune des deux séries de lames 11, 12 puisse être placée d'un côté axial de cette valve.

Ces tubes 5 à 7 comprennent également des ouvertures distales latérales (non représentées) pour permettre au sang de rejoindre le conduit corporel, ces ouvertures étant aménagée de manière à ce que la longueur de cathéter 2 traversée par le sang soit la plus courte possible, c'est-à-dire immédiatement après, dans le sens distal le filtre 14.

Le ballonnet 13 est placé sur la face extérieure du tube 7, distalement par rapport à la série 12. Ce ballonnet 13 présente une forme annulaire et est conformé pour pouvoir occuper une position de repliage dans laquelle il présente une section telle qu'il ne fait pas obstacle à l'introduction et au coulissement du dispositif 1 dans ledit conduit corporel, et une position de dépliage, dans laquelle il occupe l'ensemble de l'espace existant entre la face extérieure du tube 7 et la paroi dudit conduit corporel et vient, par un bord périphérique 13a qu'il comprend, en appui contre cette paroi.

Le filtre 14 est placé distalement par rapport au ballonnet 13, sur le tube 7, auquel il est fixé axialement. Ce filtre 14 est en matériau souple, par exemple en résille de polyester, et est conformé pour pouvoir occuper une position de repliage dans laquelle il présente une section telle qu'il ne fait pas obstacle à l'introduction et au coulissement du dispositif 1 dans ledit conduit corporel, et une position de dépliage dans laquelle il occupe l'ensemble de l'espace existant entre la face extérieure du cathéter 2 et la paroi de ce conduit et vient, par un bord périphérique 14a qu'il comprend, en appui contre cette paroi.

Un ballonnet gonflable 35 est placé entre le tube 7 et le filtre 14 pour, selon qu'il est gonflé ou dégonflé, amener le filtre 14 dans ses positions respectives de dépliage et de repliage.

En pratique, ainsi que le montrent les figures 5 à 9, le dispositif 1 est introduit par voie percutanée dans ledit conduit corporel 50 et est coulissé dans ce conduit 50 jusqu'à ce que chacune des séries 11, 12 de lames soit placée d'un côté de la valve native 55 à traiter (figure 5). Cette position est repérée à l'aide des repères précités.

Dans cette position du dispositif, la partie proximale du cathéter 2 est située dans le coeur, préférentiellement dans le ventricule gauche, tandis que les ouvertures latérales distales précitées sont placées dans un vaisseau artériel périphérique, préferentiellement dans l'aorte ascendante.

Les ballonnets 13 et 35 sont gonflés de manière à amener le sang à s'écouler uniquement au travers du conduit 15 et à empêcher le reflux du sang lors de l'ablation de la valve 55. Un système de perfusion périphérique est mis en place pour faciliter cet écoulement.

Les lames 30 des deux séries 11, 12 sont alors déployées (figure 6) par gonflage des ballonnets 31, puis ces deux séries 11, 12 sont rapprochées l'une de l'autre par coulissement du tube 6 par rapport au tube 7, jusqu'à sectionner la valve 55 (figure 7).

Les lames 30 sont ensuite ramenées en position de repliage par dégonflage des ballonnets 31 tout en restant dans leur position de rapprochement, ce qui permet de maintenir la valve 55 sectionnée entre elles.

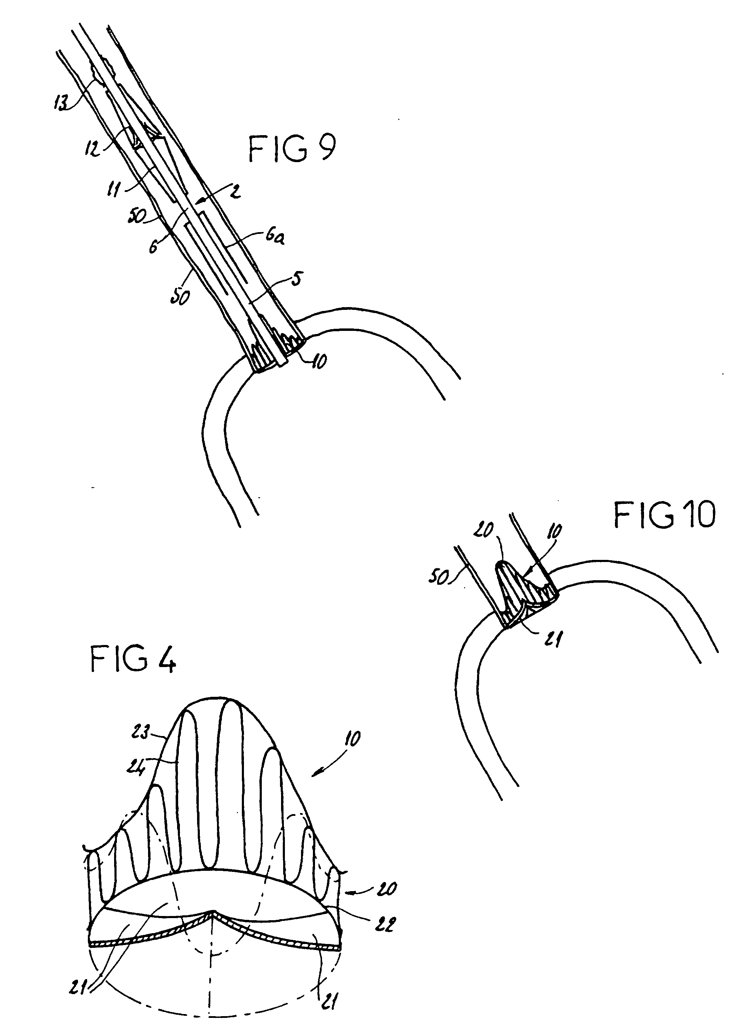

Le dispositif 1 est alors coulissé axialement dans la direction distale afin d'amener la tulipe 6a au niveau adéquat dans le conduit 50 (figure 8), après quoi la valve 10 est déployée par coulissement du tube 6 par rapport au tube 5 (figure 9).

Les ballonnets 13 et 35 sont dégonflés puis le dispositif 1 est retiré et la valve 55 sectionnée est récupérée (figure 10).

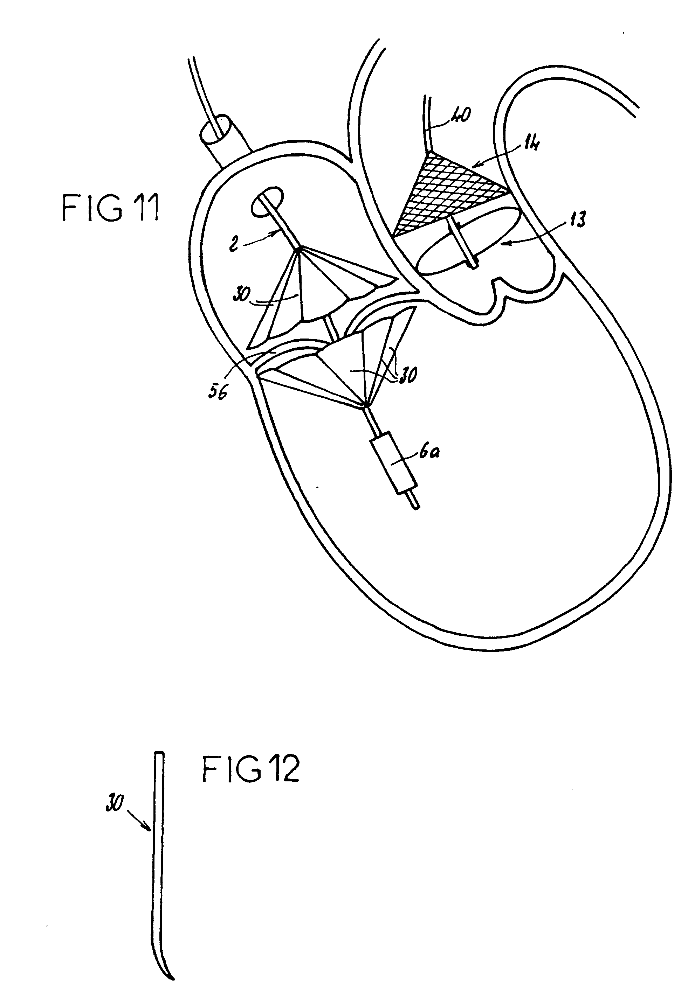

La figure 11 montre une deuxième forme de réalisation du dispositif 1, permettant une intervention sur une valve mitrale 56.

Les mêmes numéros de références sont utilisés pour désigner les mêmes éléments ou parties que précités, dès lors que ces éléments ou parties sont identiques ou similaires entre les deux formes de réalisation.

Dans ce cas, le cathéter tubulaire est remplacé par un fil de support 2, sur lequel l'une des séries de lames est montée et par un tube engagé et pouvant coulisser sur ce fil, sur lequel l'autre des séries de lames est montée ; les conduits de gonflage des ballonnets 31 cheminent le long de ces fil de support et tube ; le ballonnet 13 et le filtre 14 sont séparés du dispositif 1 et sont introduits dans l'aorte par voie artérielle périphérique, au moyen d'un fil de support 40, le long duquel cheminent les conduits de gonflage des ballonnets 13 et 35. Le dispositif 1, dépourvu de ballonnet 13 et de filtre 14, est quant à lui introduit dans le coeur par le système veineux périphérique, jusqu'à l'oreillette droite puis dans l'oreillette gauche à travers le septum inter-auriculaire, jusqu'au niveau de la valve 56.

Pour le reste, le dispositif 1 fonctionne de la même manière que précité.

L'invention fournit ainsi un dispositif de remplacement d'une valve cardiaque par voie percutanée, permettant de remédier aux inconvénients des techniques antérieures. En effet, le dispositif 1 donne toute satisfaction en ce qui concerne l'exérèse de la valve 55, 56, en permettant d'intervenir sans arrêt cardiaque et de prévenir, grâce au filtre 14, toute diffusion dans le système circulatoire de fragments de la valve 55, 56.

Il va de soi que l'invention n'est pas limitée à la forme de réalisation décrite ci-dessus à titre d'exemple mais qu'elle en embrasse, au contraire, toutes les variantes de réalisation dans le cadre des revendications ci-dessous. Ainsi, le dispositif peut comprendre un quatrième tube, engagé et pouvant coulisser sur le tube 7, ce quatrième tube comprenant le ballonnet et le filtre montés sur lui et permettant de déplacer lesdites séries de lames dans le sens axial indépendamment dudit ballonnet et/ou dudit filtre ; les lames peuvent être rectilignes comme représentées au dessin ou être courbées vers l'axe du dispositif au niveau de leur extrémité comprenant le bord tranchant, de manière à éliminer tout risque de lésion à la paroi du conduit corporel, comme montré à la figure 12 ; le filtre 14 peut être du type auto-expandable et être normalement maintenu en position rétracté par un tube coulissant, qui le recouvre, ce qui rend le ballonnet 35 inutile.

## Revendications

1. Dispositif (1) de remplacement par voie percutanée d'une valve cardiaque (55, 56) se trouvant dans un conduit corporel (50), comprenant :
- un élément allongé (2) de support ;
- une première série (11) d'éléments allongés (30), disposés autour de la circonférence dudit élément allongé (2) ; ces éléments (30) sont reliés de manière pivotante audit élément allongé (2) au niveau de leurs extrémités longitudinales proximales ; ces éléments (30) peuvent pivoter par rapport audit élément allongé (2) entre une position de repliage, dans laquelle ils se trouvent près de la paroi de l'élément allongé (2) de telle sorte qu'ils ne font pas obstacle à l'introduction et au coulissement du dispositif (1) dans le conduit corporel (50) dans lequel se trouve la valve (55, 56), en particulier dans l'aorte, et une position de dépliage, dans laquelle ces éléments (30) sont déployés ;
- une deuxième série (12) d'éléments (30), disposée consécutivement à ladite première série (11) d'éléments dans la direction distale ; les éléments (30) de cette deuxième série (12) d'éléments ont une structure identique à celle des éléments (30) de ladite première série (11) d'éléments, sinon que ces éléments (30) de cette deuxième série (12) sont reliés audit élément allongé (2) par leurs extrémités longitudinales distales ;
- des moyens (31) permettant d'amener les éléments (30) desdites première et deuxième séries (11, 12) d'éléments de leur position de repliage à leur position de dépliage ; et
- des moyens (6, 7) permettant de déplacer axialement lesdites séries (11, 12) d'éléments en direction l'une de l'autre, entre une position d'éloignement mutuel de ces séries (11, 12) d'éléments, dans laquelle une série (11) d'éléments peut être placée axialement d'un côté de la valve cardiaque native (55, 56) tandis que l'autre série (12) d'éléments est placée axialement de l'autre côté de cette valve (55, 56), et une position de rapprochement ;
**caractérisé en ce que** :
- lesdits éléments allongés (30) de ladite première série (11) sont des lames présentant chacune un bord tranchant (30a) au niveau de son extrémité longitudinale distale ;
- lesdits éléments allongés (30) de ladite deuxième série (12) sont des lames présentant chacune un bord tranchant (30b) au niveau de son extrémité longitudinale proximale ;
- dans leur position de dépliage, ces lames (30) sont déployées en corolle de telle sorte que leurs bords tranchants (30a, 30b) sont placés dans le prolongement les uns des autres et constituent ainsi des bords circulaires tranchants ;
- dans la position de rapprochement des deux séries (11, 12) de lames (30), lesdits bords circulaires tranchants sont amenés en contact mutuel pour sectionner la valve cardiaque native (55, 56) de manière à la séparer dudit conduit corporel (50) ; et
- le dispositif (1) comprend des moyens de repérage par voie percutanée de la position axiale du dispositif (1) par rapport à la valve cardiaque native (55, 56), permettant de positionner chacune des deux séries (11, 12) de lames (30) d'un côté de cette valve.

2. Dispositif (1) selon la revendication 1, **caractérisé en ce qu'**il comprend une valve prothétique proximale (10), à structure radialement déployable, cette valve prothétique (10) pouvant occuper une position de repliage, dans laquelle elle se trouve près de la paroi dudit élément allongé (2) et ne fait pas obstacle à l'introduction et au coulissement du dispositif (1) dans ledit conduit corporel (50), et une position de dépliage, dans laquelle elle prend appui contre la paroi de ce conduit (50) et est à même de remplacer la valve cardiaque native (55, 56).

3. Dispositif (1) selon la revendication 1 ou la revendication 2, **caractérisé en ce que** ledit élément allongé de support est un cathéter tubulaire (2).

4. Dispositif (1) selon l'une des revendications 1 à 3, **caractérisé en ce qu'**il comprend un ballonnet gonflable distal (13), placé au niveau de la face extérieure dudit élément allongé (2) ; ce ballonnet (13) est conformé pour pouvoir occuper une position de repliage, dans laquelle il présente une section telle qu'il ne fait pas obstacle à l'introduction et au coulissement du dispositif (1) dans ledit conduit corporel (50), et une position de dépliage, dans laquelle il occupe l'ensemble de l'espace existant entre la face extérieure dudit élément allongé (2) et la paroi dudit conduit corporel (50) et vient, par un bord périphérique (13a) qu'il comprend, en appui contre cette paroi.

5. Dispositif (1) selon l'une des revendications 1 à 4, **caractérisé en ce qu'**il comprend un filtre distal (14) en matériau souple, placé au niveau de la face extérieure dudit élément allongé (2) ; ce filtre (14) est conformé pour pouvoir occuper une position de repliage, dans laquelle il présente une section telle qu'il ne fait pas obstacle à l'introduction et au coulissement du dispositif (1) dans ledit conduit corporel (50), et une position de dépliage, dans laquelle il occupe l'ensemble de l'espace existant entre la face extérieure dudit élément allongé (2) et la paroi de ce conduit (50) et vient, par un bord périphérique (14a) qu'il comprend, en appui contre cette paroi.

6. Dispositif (1) selon la revendication 4 ou la revendication 5, **caractérisé en ce qu'**il comprend des moyens permettant de déplacer lesdites séries de lames dans le sens axial indépendamment dudit ballonnet et/ou dudit filtre.

7. Dispositif (1) selon la revendication 4 ou la revendication 5, **caractérisé en ce que** ledit ballonnet (13) et/ou ledit filtre (14) sont montés sur un élément allongé de support qui leur est propre, et sont séparés du dispositif (1).

8. Dispositif (1) selon l'une des revendications 2 à 7, **caractérisé en ce que** la valve prothétique (10) comprend une armature (20) en matériau à mémoire de forme, notamment en alliage Nickel-titane connu sous la dénomination "NITINOL".

9. Dispositif (1) selon l'une des revendications 2 à 8, **caractérisé en ce que** la valve prothétique (10) comprend des valvules (21) en matériau biologique (valvules animales ou humaines conservées), ou des valvules en matériau synthétique, tel qu'en polymère.

10. Dispositif (1) selon l'une des revendications 1 à 9, **caractérisé en ce que** les lames sont courbées vers l'axe du dispositif au niveau de leur extrémité comprenant le bord tranchant.

## Claims

1. A device (1) for replacing, via a percutaneous route, a heart valve (55, 56) located in a bodily vessel (50), comprising :
- an elongate support element (2) ;
- a first series (11) of elongate members (30), arranged around the circumference of said elongate element (2) ; these members (30) are pivotably connected to said elongate element (2) at their proximal longitudinal end ; these members (30) can pivot with respect to said elongate element (2) between a furled position in which they are near to the wall of the elongate element (2) so that they do not impede the introduction and sliding of the device (1) into and in the bodily vessel (50) in which the valve (55, 56) is located, particularly within the aorta, and an unfurled position in which these members (30) are deployed ;
- a second series (12) of members (30) which is arranged after said first series (11) of members in the distal direction ; the members (30) of this second series (12) of members have a structure identical to that of the members (30) of said first series (11) of members except that these members (30) of this second series (12) are connected to said elongate element by their distal longitudinal end ;
- means (31) for bringing the members (30) of said first and second series (11, 12) of members from their furled position to their unfurled position ; and
- means (6, 7) for moving said series (11, 12) of members axially toward one another, between a position in which these series (11, 12) of members are away from each other and in which one series (11) of members can be placed axially on one side of the native cardiac valve (55, 56) while the other series (12) of members is placed axially on the other side of this valve (55, 56), and a close-together position ;
**characterized in that**
- said elongated members (30) of said first series (11) are blades each having a cutting edge (30a) at its distal longitudinal end ;
- said elongated members (30) of said second series (12) are blades each having a cutting edge (30b) at its proximal longitudinal end ;
- in their unfurled position, these blades (30) are deployed into a corolla in such a way that their cutting edges (30a, 30b) are placed in the continuation of one another and thus constitute a circular cutting edge ;
- in said close-together position of said series (11, 12) of blades (30), said circular cutting edges are brought into mutual contact for cutting through the native cardiac valve (55, 56) so as to detach it from said bodily vessel (50)
- the device (1) comprises means of identifying, by a percutaneous route, the axial position of the device (1) with respect to the cardiac native valve (55, 56) so as to position each of the two aforementioned series (11, 12) of blades (30) on one side of this valve.

2. The device (1) as claimed in claim 1, **characterized in that** it comprises a proximal prosthetic valve (10), with a radially deployable structure, it being possible for this prosthetic valve (10) to occupy a furled position in which it is near the wall of said elongate element (2) and does not impede the introduction and sliding of the device (1) into and in said bodily vessel (50), and an unfurled position in which it bears against the wall of this vessel (50) and is able to replace the native heart valve (55, 56).

3. The device (1) as claimed in claim 1 or claim 2, **characterized in that** said elongate support element is a tubular catheter (2).

4. The device (1) as claimed in one of claims 1 to 3, **characterized in that** it comprises a distal inflatable balloon (13), placed at the exterior face of said elongate element (2) ; this balloon (13) is shaped to be able to occupy a furled position in which it has a cross section such that it does not impede the sliding introduction of the device (1) into said bodily. vessel (50), and an unfurled position in which it occupies all of the space between the exterior face of said elongate element (2) and the wall of said bodily vessel (50) and, via a peripheral edge (13a) that it comprises, bears against this wall.

5. The device (1) as claimed in one of claims 1 to 4, **characterized in that** it comprises a distal filter (14) made of flexible material placed at the exterior face of said elongate element (2) ; this filter (14) is shaped to be able to occupy a furled position in which it has a cross section such that it does not impede the introduction and sliding of the device (1) into and in said bodily vessel (50), and an unfurled position in which it occupies all of the space between the exterior face of said elongate element (2) and the wall of this vessel (50) and, via a peripheral edge (14a) that it comprises, bears against this wall.

6. The device (1) as claimed in claim 4 or claim 5, **characterized in that** it comprises means for moving said series of blades in the axial direction independently of said balloon and/or of said filter.

7. The device (1) as claimed in claim 4 or claim 5, **characterized in that** said balloon (13) and/or said filter (14) are mounted on an elongate support element specific to them and are separate from the device (1).

8. The device (1) as claimed in one of claims 2 to 7, **characterized in that** the prosthetic valve (10) comprises an armature (20) made of a shape memory material, particularly a nickel-titanium alloy known by the name of "NITINOL".

9. The device (1) as claimed in one of claims 2 to 8, **characterized in that** the prosthetic valve (10) comprises valve leaflets (21) made of biological material (preserved animal or human valve leaflets) or valve leaflets made of a synthetic material such as a polymer.

10. The device (1) as claimed in one of claims 1 to 9, **characterized in that** the blades are curved toward the axis of the device at their end that has the cutting edge.

## Patentansprüche

1. Vorrichtung (1) für den Ersatz einer Herzklappe (55, 56), die sich in einem Körperkanal (50) befindet, auf perkutanem Weg, umfassend:
- ein längliches Stützelement (2);
- eine erste Reihe (11) von länglichen Elementen (30), die um den Umfang des länglichen Elements (2) angeordnet sind; wobei diese Elemente (30) schwenkbar mit dem länglichen Element (2) im Bereich ihrer proximalen Längsenden verbunden sind; wobei diese Elemente (30) in Bezug auf das längliche Element zwischen einer zurückgeklappten Position, in der sie sich in der Nähe der Wand des länglichen Elements (2) befinden, so dass sie kein Hindernis für die Einführung und das Gleiten der Vorrichtung (1) in dem Körperkanal (50), in dem sich die Klappe (55, 56) befindet, insbesondere der Aorta, darstellen, und einer ausgeklappten Position, in der diese Elmente (30) ausgefahren sind, schwenken können;
- eine zweite Reihe (12) von Elementen (30), die im Anschluss an die erste Reihe (11) von Elementen in distaler Richtung angeordnet ist; wobei die Elemente (30) dieser zweiten Reihe (12) von Elementen eine identische Struktur wie jene der Elemente (30) der ersten Reihe (11) von Elementen aufweisen, außer dass diese Elemente (30) dieser zweiten Reihe (12) mit dem länglichen Element (2) mit ihren distalen Längsenden verbunden sind;
- Mittel (31), die es ermöglichen, die Elemente (30) der ersten und der zweiten Reihe (11, 12) von Elementen von ihrer zurückgeklappten Position in ihre ausgeklappte Position zu bringen; und
- Mittel (6, 7), die es ermöglichen, die Reihen (11, 12) von Elementen in Richtung zueinander zwischen einer Position der wechselseitigen Entfernung dieser Reihen (11, 12) von Elementen, in der eine Reihe (11) von Elementen axial auf einer Seite der nativen Herzklappe (55, 56) angeordnet werden kann, während die andere Reihe (12) von Elementen axial auf der anderen Seite dieser Klappe (55, 56) angeordnet ist, und einer Position der Annäherung axial zu verschieben;
**dadurch gekennzeichnet, dass**:
- die länglichen Elemente (30) der ersten Reihe (11) Blättchen sind, die jeweils einen scharfen Rand (30a) im Bereich ihres distalen Längsendes aufweisen;
- die länglichen Elemente (30) der zweiten Reihe (12) Blättchen sind, die jeweils einen scharfen Rand (30b) im Bereich ihres proximalen Längsendes aufweisen;
- in ihrer aufgeklappten Position diese Blättchen (30) in Form einer Blütenkrone aufgebreitet werden, so dass ihre scharfen Ränder (30a, 30b) in der Verlängerung zueinander angeordnet sind und so kreisförmige scharfe Ränder darstellen;
- in der Position der Annäherung der beiden Reihen (11, 12) von Blättchen (30) die kreisförmigen scharfen Ränder in wechselseitigen Kontakt gebracht werden, um die native Herzklappe (55, 56) abzuschneiden, um sie von dem Körperkanal (50) zu trennen; und
- die Vorrichtung (1) Mittel zur Erfassung der Axialposition der Vorrichtung (1) in Bezug auf die native Herzklappe (55, 56) auf perkutanem Weg umfasst, die es ermöglichen, jede der beiden Reihen (11, 12) von Blättchen (30) auf einer Seite dieser Klappe anzuordnen.

2. Vorrichtung (1) nach Anspruch 1, **dadurch gekennzeichnet, dass** sie eine proximale Protheseklappe (10) mit radial aufbreitbarer Struktur umfasst, wobei diese Protheseklappe (10) eine zurückgeklappte Position, in der sie sich nahe der Wand des länglichen Elements (2) befindet und kein Hindernis für die Einführung und das Gleiten der Vorrichtung (1) in dem Körperkanal (50) darstellt, und eine ausgeklappte Position einnehmen kann, der sie an der Wand dieser Leitung (50) anliegt und die native Herzklappe (55, 56) ersetzen kann.

3. Vorrichtung (1) nach Anspruch 1 oder Anspruch 2, **dadurch gekennzeichnet, dass** das längliche Stützelement ein röhrenförmiger Katheter (2) ist.

4. Vorrichtung (1) nach einem der Ansprüche 1 bis 3, **dadurch gekennzeichnet, dass** sie einen distalen aufblasbaren Ballon (13) umfasst, der in der Nähe der Außenfläche des länglichen Elements (2) angeordnet ist; wobei dieser Ballon (13) derart ausgeführt ist, dass er eine zurückgeklappte Position, in der er einen derartigen Querschnitt aufweist, dass er kein Hindernis für die Einführung und das Gleiten der Vorrichtung (1) in dem Körperkanal (50) darstellt, und eine ausgeklappte Position einnehmen kann, in der er den gesamten vorhandenen Raum zwischen der Außenfläche des länglichen Elements (2) und der Wand des Körperkanals (50) einnimmt und mit einem Umfangsrand (13a), den er umfasst, an dieser Wand anliegt.

5. Vorrichtung (1) nach einem der Ansprüche 1 bis 4, **dadurch gekennzeichnet, dass** sie einen distalen Filter (14) aus weichem Material umfasst, der im Bereich der Außenfläche des länglichen Elements (2) angeordnet ist; wobei dieser Filter (14) derart ausgeführt ist, um eine zurückgeklappte Position, in der er einen derartigen Querschnitt aufweist, dass er kein Hindernis für die Einführung und das Gleiten der Vorrichtung (1) in dem Körperkanal (50) darstellt, und eine ausgeklappte Position einzunehmen, in der er den gesamten vorhandenen Raum zwischen der Außenfläche des länglichen Elements (2) und der Wand dieser Leitung (50) einnimmt und mit einem Umfangsrand (14a), den er umfasst, an dieser Wand anliegt.

6. Vorrichtung (1) nach Anspruch 4 oder Anspruch 5, **dadurch gekennzeichnet, dass** sie Mittel umfasst, die es ermöglichen, die Reihen von Blättchen in Axialrichtung unabhängig von dem Ballon und/oder Filter zu verschieben.

7. Vorrichtung (1) nach Anspruch 4 oder Anspruch 5, **dadurch gekennzeichnet, dass** der Ballon (13) und/oder der Filter (14) auf einem länglichen Stützelement, das ihnen eigen ist, befestigt und von der Vorrichtung (1) getrennt sind.

8. Vorrichtung (1) nach einem der Ansprüche 2 bis 7, **dadurch gekennzeichnet, dass** die Protheseklappe (10) einen Beschlag (20) mit Formgedächtnis, insbesondere aus einer Nickel-Titan-Legierung, die unter der Bezeichnung "NITINOL" bekannt ist, umfasst.

9. Vorrichtung (1) nach einem der Ansprüche 2 bis 8, **dadurch gekennzeichnet, dass** die Protheseklappe (10) Klappen (21) aus biologischem Material (konservierte tierische oder menschliche Klappen) oder Klappen aus synthetischem Material, wie beispielsweise aus Polymer, umfasst.

10. Vorrichtung (1) nach einem der Ansprüche 1 bis 9, **dadurch gekennzeichnet, dass** die Blättchen zur Achse der Vorrichtung im Bereich ihres den scharfen Rand umfassenden Endes gekrümmt sind.
